(19) Europäisches Patentamt
European Patent Office
Office européen des brevets

(11) Publication number : **0 307 106 B1**

(12) **EUROPEAN PATENT SPECIFICATION**

(45) Date of publication of patent specification : **15.01.92 Bulletin 92/03**

(51) Int. Cl.$^5$ : **C07C 43/23,** C07C 41/26

(21) Application number : **88307796.8**

(22) Date of filing : **23.08.88**

# (54) P- or m-tert butoxyphenethyl alcohol and process for preparing the same.

(30) Priority : **11.09.87 JP 226570/87**

(43) Date of publication of application : **15.03.89 Bulletin 89/11**

(45) Publication of the grant of the patent : **15.01.92 Bulletin 92/03**

(84) Designated Contracting States : **CH DE FR GB LI SE**

(56) References cited : **CHEMICAL ABSTRACTS, vol. 52, no. 15, 10th August 1958, no. 12803h-12804c, Columbus, Ohio, US; G.S. KOLESNIKOV et al.: "Carbon chain polymers and copolymers. I. Synthesis and polymerization of 4-alkoxystyrenes",**

(73) Proprietor : **HOKKO CHEMICAL INDUSTRY CO., LTD**
**4-20, 4-chome, Nihonbashi-Hongoku-cho**
**Chuo-ku**
**Tokyo (JP)**

(72) Inventor : **Umeno, Masayuki**
**521-3 Chigasaki**
**Chigasaki-shi Kanagawa-ken (JP)**
Inventor : **Araki, Yoshihiro**
**c/o Hokko Kagaku-Ryo 2385, Toda**
**Atsugi-shi, Kanagawa-ken (JP)**

(74) Representative : **Cresswell, Thomas Anthony et al**
**J.A. Kemp & Co. 14 South Square Gray's Inn**
**London WC1R 5EU (GB)**

**EP 0 307 106 B1**

Note : Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

1) Field of the Invention

The present invention relates to novel para- or meta-tert-butoxyphenethyl alcohol and a process for preparing the same.

The compound of the invention is useful as an intermediate for the synthesis of pharmaceuticals, agrochemicals and polymers, for example, a cardiac drug metoprolol.

2) Description of Prior Art

A large number of alkoxyphenethyl alcohols have heretofore been known.

For example, there is described in Chemical Abstracts Vol. 52, page 12803 (1958) that p-n-butoxyphenethyl alcohol which is an isomer of the compound of the invention is produced by reacting a p-n-butoxyphenylmagnesium halide with ethylene oxide. The compound of the invention, however, is a novel compound which has not been described in literatures.

Production of hydroxyphenethyl alcohols by dealkylation reaction from n-alkoxyphenethyl alcohols such as normal butoxyphenethyl alcohol requires a treatment under severe conditions such as with hydrobromic acid, hydroiodic acid or trifluoroacetic acid and is associated with reduction in yield and purity. Particularly, de-n-butylation reaction from n-butoxyphenethyl alcohol is encountered with great difficulties as shown in Referential Examples below. Whereas protection by benzyl group as in benzyloxyphenethyl alcohol is a general method widely employed, debenzylation reaction which is an elimination reaction by means of reduction with hydrogen meets several operational troubles such as side reactions on other functional groups and dangers during operation. Moreover, compounds comprising protection by trimethylsilyl group or dimethyl-tert-butylsilyl group as a protective group as in trimethylsilyloxyphenethyl alcohol or dimethyl-tert-butylsilyloxyphenethyl alcohol have such disadvantages that the protective group undesirably react with Grignard reagent or these compounds cost high.

Summary of the Invention

As a result of extensive studies for solving the problems associated with the prior art, we have found that tert-butyl group is very well suitable as a protective group of the hydroxyl group in the synthesis of p- or m-hydroxyphenethyl alcohol and a derivative thereof.

According to the present invention, there is provided p- or m-tert-butoxyphenethyl alcohol represented by the formula

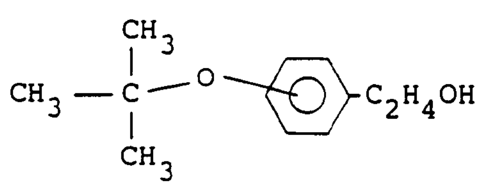

Further, according to the invention, there is provided a process for preparing p- or m-tert-butoxyphenethyl alcohol which comprises reacting a p- or m-tert-butoxyphenyl halide with metallic magnesium to produce a p- or m-tert-butoxyphenylmagnesium halide and reacting the same with ethylene oxide.

Detailed Description of the Invention

The process for preparing p- or m-tert-butoxyphenethyl alcohol according to the present invention is shown by the following reaction scheme :

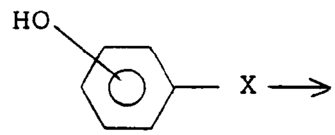

( II )

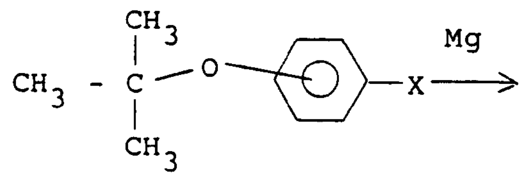


( III )

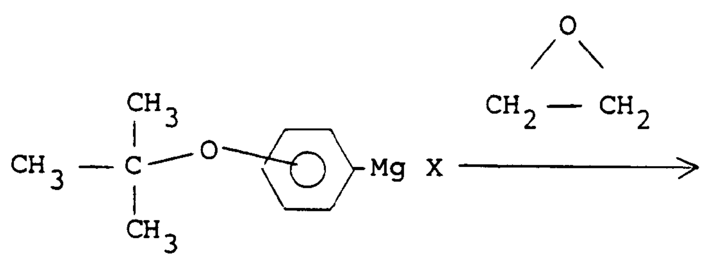

( IV )

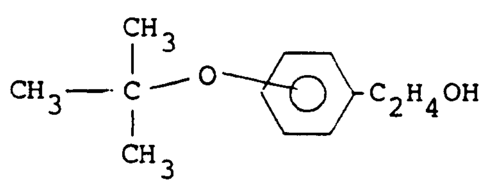

( I )

wherein X represents a halogen atom and a hydroxyl group or tert-butoxy group is bonded at para- or meta-position.

p- or m-tert-Butoxyphenethyl alcohol (I) of the invention can be prepared by reacting a p- or m-tert-butoxyphenyl halide (III) in which hydroxyl group of p- or m-halogenophenol (II) is protected by tert-butyl group is reacted with metallic magnesium to convert the former to a Grignard reagent, a p- or m-tert-butoxyphenylmagnesium halide (IV) and reacting the same with ethylene oxide. The process for preparing the compound of the invention will be described in more details below.

(a) Preparation of a p- or m-tert-butoxyphenyl halide (compound of the general formula (III))

A compound of the general formula (III) is produced by reacting a compound of the general formula (II)

with isobutylene in an aprotic solvent, i.e., in a halogenated hydrocarbon solvent system such as dichloromethane or dichloroethane or in an aromatic hydrocarbon system such as benzene or toluene in the presence of a catalyst such as sulfuric acid, p-toluenesulfonic acid, methanesulfonic acid, boron trifluoride or a strong acid ion-exchange resin.

(b) Preparation of a p- or m-tert-butoxyphenylmagnesium halide (compound of the general formula (IV))

A compound of the general formula (IV), a Grignard reagent, is produced by dropwise adding a compound of the general formula (III) to a stirred mixture of activated metallic magnesium in anhydrous tetrahydrofuran at a temperature from 40°C to refluxing temperature of the solvent used and stirring continuously for additional 1-8 hours.

The metallic magnesium used in the reaction is in the form of tapes or flakes and is used in a proportion of about 1-2 moles per mole of the compound of the general formula (III). In order to activate the metallic magnesium prior to initiation of the reaction, it is stirred in the atmosphere of nitrogen or under reduced pressure or reacted with a little amount of such a reagent as iodide, methyl iodide, methyl bromide or dibromoethane. Such treatment is effective for smoothly progressing the Grignard reaction. In the preparation of the compound of the general formula (IV) the solvent can be tetrahydrofuran or diethyl ether used alone or in mixture with benzene or toluene with the same results produced.

p- or m-tert-Butoxyphenylmagnesium chloride which is a compound of the general formula (IV) wherein X is chlorine is a novel compound.

(c) Preparation of p- or m-tert-butoxyphenethyl alcohol (compound of the formula (I))

A compound of the formula (I) is produced by reacting the Grignard reagent (IV) with ethylene oxide. Ethylene oxide used in an amount of 1-2 moles per mole of the Grignard reagent (IV) is bubbled in gaseous form or dropped in dilution with a solvent at 0-50°C, preferably 20-30°C followed by stirring for 1-5 hours.

After completion of the reaction, an aqueous solution of ammonium chloride or a diluted aqueous solution of a mineral acid is poured to separate the organic layer which is then washed with water and dried over sodium sulfate. The solvent is distilled off and the residue is subjected to distillation under reduced pressure to give the desired compound of the general formula (I)

The invention will be described in more particulars with reference to Examples and Referential Examples.

Example 1

In a one-liter four-necked flask equipped with a stirrer and a reflux condenser, purged with nitrogen and dried was placed 29.2 g (1.2 mol.) of metallic magnesium flakes to which were added 50 ml of anhydrous tetrahydrofuran and 2 ml of ethyl bromide. After confirming initiation of the reaction by bubbling on the surface of the metallic magnesium, a solution of 184.6 g (1.0 mol.) of p-tert-butoxychlorobenzene in 500 ml of anhydrous tetrahydrofuran was added dropwise at the refluxing temperature over approximately 2 hours followed by stirring for 5 hours to give p-tert-butoxyphenylmagnesium chloride.

When an aliquot portion of the reaction mixture was taken, decomposed by pouring water and analyzed by gas chromatography, conversion ratio of the Grignard reagent was found to be 99.8%.

Subsequently, 48.4 g (1.1 mol.) of ethylene oxide was introduced at 20-30°C over 1 hour, and then stirring was continued at the same temperature for 2 hours.

The reaction mixture was then poured into 500 ml of saturated aqueous solution of ammonium chloride, followed by addition of 500 ml of toluene. The organic layer was separated, washed with water and dried over anhydrous sodium sulfate. The solvent was distilled off, and the residue was subjected to distillation under reduced pressure to give 175.4 g (yield 90.3%) of p-tert-butoxyphenethyl alcohol as distillate boiling at 117-121°C/3 mmHg. Purity as determined by gas chromatography was 99.8%.

Analysis and physicochemical properties were as follows :

(1) Elementary analysis

| | C (%) | H (%) |
|---|---|---|
| Calc'd. | 74.2 | 9.3 |
| Found | 74.3 | 9.3 |

(2) NMR spectrum

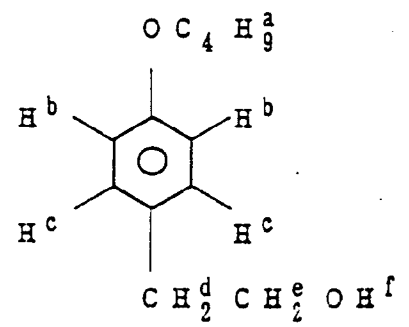

δ 1.30 (9H,s,$H^a$) δ 2.74 (2H,t,$H^d$)

δ 3.10 (1H,s,$H^f$) δ 3.68 (2H,t,$H^e$)

δ 6.88 (2H,d,$H^b$) δ 7.18 (2H,d,$H^c$)

(3) Infrared absorption spectrum

| | |
|---|---|
| 3350cm$^{-1}$ | O-H stretching |
| 1100cm$^{-1}$ | |

Example 2

In a one-liter four-necked flask equipped with a stirrer and a reflux condenser, purged with nitrogen and dried was placed 24.3 g (1.0 mol.) of metallic magnesium flakes to which were added 10 ml of anhydrous diethyl ether and 2 ml of ethyl bromide. After confirming initiation of the reaction by bubbling on the surface of the metallic magnesium, a solution of 114.6 g (0.5 mol.) of p-tert-butoxybromobenzene in 400 ml of anhydrous diethyl ether was added dropwise at the refluxing temperature over approximately 1 hour, and stirring was continued for 5 hours to give p-tert-butoxyphenylmagnesium bromide. An aliquot portion of the reaction mixture was taken, decomposed by pouring water and analyzed by gas chromatography to find that conversion ratio of the Grignard reagent was 99.9%.

Subsequently, 24.2 g (0.55 mol.) of ethylene oxide was introduced at 20-30°C over 1 hour, and stirring was continued at the same temperature for 2 hours to prepare p-tert-butoxyphenethyl alcohol. Then, the reaction mixture was poured into 250 ml of saturated aqueous solution of ammonium chloride, followed by addition of 250 ml of toluene. The organic layer was separated, washed with water and then dried over anhydrous sodium sulfate. The solvent was distilled off, and the residue was subjected to distillation under reduced pressure to give 82.9 g (yield 85.3%) of p-tert-butoxyphenethyl alcohol as distillate boiling at 117-121°C/3 mmHg. Purity as determined by gas chromatography was 99.5%.

Example 3

In a one-liter four-necked flask equipped with a stirer and a reflux condenser, purged with nitrogen and dried was placed 29.2 g (1.2 mol.) of metallic magnesium flakes to which were added 50 ml of anhydrous tetrahydrofuran and 2 ml of ethyl bromide. After confirming initiation of the reaction by bubbling on the surface of the metallic magnesium, a solution of 184.6 g (1.0 mol.) of m-tert-butoxychlorobenzene in 500 ml of anhydrous tetrahydrofuran was added dropwise at the refluxing temperature over approximately 2 hours, and stirring was continued for 5 hours to give m-tert-butoxyphenylmagnesium chloride.

An aliquot portion of the reaction mixture was taken, hydrolyzed and analyzed by gas chromatography to find that conversion ratio of the Grignard reagent was 99.9%.

Subsequently, 48.4 g (1.1 mol.) of ethylene oxide was introduced at 20-30°C over 1 hour, and stirring was continued at the same temperature for 2 hours.

Then, the reaction mixture was poured into 500 ml of saturated aqueous solution of ammonium chloride, followed by addition of 500 ml of toluene. The organic layer was separated, washed with water and dried over anhydrous sodium sulfate. The solvent was distilled off, and the residue was subjected to distillation under reduced pressure to give 176.2 g (yield 90.7%) of m-tert-butoxyphenethyl alcohol as distillate boiling at 129-130°C/4 mmHg.

Purity as determined by gas chromatography was 99.9%.

Analysis and physicochemical properties were as follows :

(1) Elementary analysis

| | C (%) | H (%) |
|---|---|---|
| Calc'd. | 74.2 | 9.3 |
| Found | 74.2 | 9.2 |

(2) NMR spectrum

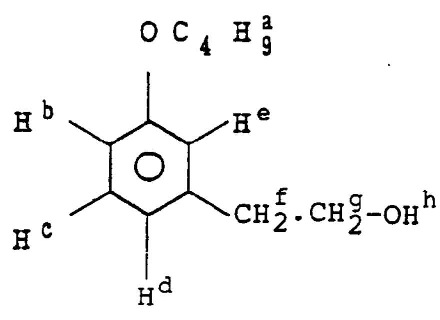

δ 1.30 (9H,s,$H^a$) δ 2.75 (2H,t,$H^f$)

δ 3.40 (1H,bs,$H^h$) δ 3.65 (2H,t,$H^g$)

δ 6.60-7.20 (4H,m,$H^b$-$H^e$)

(3) Infrared absorption spectrum

| | |
|---|---|
| 3350 $cm^{-1}$ | O-H stretching |
| 1140 $cm^{-1}$ | |

[Debutoxylation test]

Referential Example 1

In a 300-ml flask equipped with a stirrer was placed 97.1 g (0.5 mol.) of p-tert-butoxyphenethyl alcohol, to which was then dropwise added 150 g of concentrated hydrochloric acid at 25-30°C, and stirring was continued for 2 hours.

After completion of the reaction, the reaction mixture was neutralized with an aqueous solution of sodium carbonate and then extracted with 300 ml of dichloromethane. Then, the solvent was distilled off to afford crude crystals of p-hydroxyphenethyl alcohol. Subsequent distillation in vaccum yielded 63.4 g of the product as distillate boiling at 140°C/3 mmHg (purity as determined by gas chromatography 99.9%, yield 91.9%). Melting point of the desired product was 91-92°C, and the infrared absorption spectrum was completely identical with that of authentic p-hydroxyphenethyl alcohol.

On the other hand, 19.4 g (0.1 mol.) of p-n-butoxyphenethyl alcohol and 30 g (0.3 mol.) of concentrated hydrochloric acid were placed in a 100-ml flask and stirred at 25-30°C for 2 hours with no change observed.

Stirring was then continued at the refluxing temperature for additional 5 hours also with no change observed. Similarly, stirring in 30 g of concentrated hydrobromic acid at the refluxing temperature for 5 hours resulted in disappearance of the starting material but no formation of the desired p-hydroxyphenethyl alcohol. There were produced as by-products p-n-butoxyphenethyl bromide, a brominated product on the alcohol moiety, and partly p-hydroxyphenethyl bromide.

Referential Example 2

In a 300-ml flask equipped with a stirrer was placed 97.1 g (0.5 mol.) of m-tert-butoxyphenethyl alcohol, to which was then dropwise added 200 g of concentrated hydrochloric acid at 25-30°C, and stirring was continued for 2 hours.

After completion of the reaction, the reaction mixture was neutralized with an aqueous solution of sodium carbonate and extracted with 300 ml of dichloromethane. Then, the solvent was distilled off, and the residue was subjected to distillation under reduced pressure. There was obtained 64.5 g of m-hydroxyphenethyl alcohol as distillate boiling at 169-170°C/5 mmHg (purity as determined by gas chromatography 99.9%, yield 93.3%).

On the other hand, debutoxylation of m-n-butoxyphenethyl alcohol carried out in the same way as with p-n-butoxyphenethyl alcohol in Referential Example 1 resulted in no formation of the desired m-hydroxyphenethyl alcohol. There were produced as by-products m-n-butoxyphenethyl bromide, a brominated product on the alcohol moiety, and partly m-hydroxyphenethyl bromide.
(NB. /mm Hg = 1.33322 mbar)

## Claims

1. p- or m-tert-Butoxyphenethyl alcohol represented by the formula

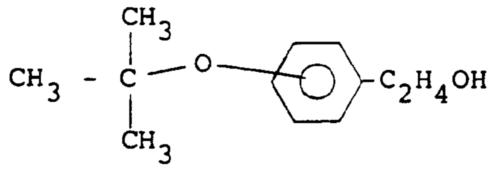

2. A process for preparing p- or m-tert-butoxyphenethyl alcohol which comprises reacting a p- or m-butoxyphenyl halide with metallic magnesium to produce a p- or m-tert-butoxyphenylmagnesium halide and reacting the same with ethylene oxide.

3. A process according to Claim 2 wherein the p- or m-tert-butoxyphenyl halide is p- or m-tert-butoxychlorobenzene.

## Patentansprüche

1. p- oder m-tert.-Butoxiphenylenethyl Alkohol dargestellt durch die Formel

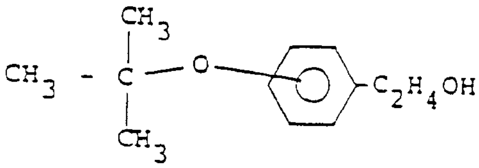

2. Verfahren zur Herstellung von p- oder m-tert.-Butoxi-phenylenethyl Alkohol durch Umsetzung eines p- oder m-Butoxiphenylhalids mit metallischem Magnesium zur Erzeugung eines p- oder m-tert.-Butoxiphenylmagnesiumhalids und Umsetzung desselben mit Ethylenoxid.

3. Verfahren nach Anspruch 2 wobei das p- oder m-tert.-Butoxiphenylhalid p- oder m-tert.-Butoxichlorobenzol ist.

## Revendications

1. Alcool p- ou m-(t-butoxy)phénéthylique ayant pour formule :

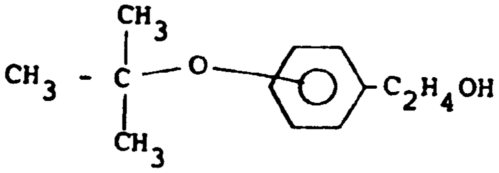

2. Procédé pour préparer l'alcool p- ou m-(t-butoxy)phénéthylique qui comprend la réaction d'un halogénure de p- ou m-butoxyphényle avec du magnésium métallique pour obtenir un halogénure de p- ou m-(t-butoxy)phényl magnésium et la réaction de ce dernier avec de l'oxyde d'éthylène.

3. Procédé selon la revendication 2, dans lequel l'halogénure de p- ou m-(t-butoxy)phényle est le p- ou m-(t-butoxy)chlorobenzène.